(19) **Europäisches Patentamt / European Patent Office / Office européen des brevets**

(11) **EP 3 357 488 B1**

(12) # EUROPEAN PATENT SPECIFICATION

(45) Date of publication and mention
of the grant of the patent:
**23.12.2020 Bulletin 2020/52**

(21) Application number: **16850640.0**

(22) Date of filing: **21.09.2016**

(51) Int Cl.:
*A61K 8/891* (2006.01)   *A61K 8/85* (2006.01)
*A61K 8/895* (2006.01)   *A61Q 1/00* (2006.01)
*A61Q 17/04* (2006.01)

(86) International application number:
**PCT/JP2016/004302**

(87) International publication number:
**WO 2017/056460 (06.04.2017 Gazette 2017/14)**

(54) **COSMETIC MATERIAL**

KOSMETIKMATERIAL

PRODUIT COSMÉTIQUE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **29.09.2015 JP 2015192054
14.03.2016 JP 2016049818**

(43) Date of publication of application:
**08.08.2018 Bulletin 2018/32**

(73) Proprietor: **Shiseido Company, Ltd.
Tokyo 104-0061 (JP)**

(72) Inventor: **KUBOTA, Shun
Yokohama-shi
Kanagawa 224-8558 (JP)**

(74) Representative: **Ter Meer Steinmeister & Partner
Patentanwälte mbB
Nymphenburger Straße 4
80335 München (DE)**

(56) References cited:
WO-A1-2006/120876   JP-A- H0 769 856
JP-A- H01 247 472   JP-A- H07 175 242
JP-A- 2010 064 986   JP-A- 2014 172 872
JP-A- 2015 086 196   JP-B2- 2 921 058
JP-B2- 3 503 814   US-A1- 2009 269 374
US-A1- 2011 150 800   US-A1- 2014 328 779
US-A1- 2015 196 467

• **Keshoryo Bun'ya ni Okeru Kochi Gijutsushu, 16
March 2012 (2012-03-16), page 1, XP009509747,**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

Technical Field

The present invention relates to a cosmetic, and more particularly relates to a cosmetic containing an elastic powder.

Background Art

In general, cosmetics contain various types of powders for the purposes of improvement of skin color, UV protection, adsorption of sweat or sebum, etc. Since it is necessary to increase uniformity and adhesion to the skin of the various types of powders when the cosmetics are applied to the skin, such cosmetics conventionally contain a high amount of non-volatile oil component and/or ester-based oil component that fits comfortably on the skin.
However, cosmetics containing the non-volatile oil component in a high amount and/or the ester-based oil component have such problems that they lose dry touch during use, which is a unique requirement for cosmetics, and such cosmetics are apt to spread unevenly during application, making them difficult to be uniformly applied to the skin. In order to address such problems, Patent Literature 1 proposes a cosmetic containing a certain amount of dimethylpolysiloxane having a specific viscosity, and a high amount of volatile oil component, such as a linear silicone having a relatively low molecular weight or a cyclic silicone.
Further, Patent Literature 2 teaches that a fluid composition that has sufficient fluidity to provide refreshing feel, and provides comfortable and easy-to-spread feel during application, while maintaining usual characteristics of water-in-oil type emulsions, can be obtained by adding a polyol alkyl ester and a silicone emulsifier to a water-in-oil type emulsion composition wherein a half of the oil phase thereof is formed by a branched hydrocarbon oil and a volatile silicone oil.

Citation List

Patent Literature

[PTL 1] Japanese Unexamined Patent Publication No. H8-259417
[PTL 2] Japanese Unexamined Patent Publication No. 2000-229838

Summary of Invention

Technical Problem

However, although conventional cosmetics are easily spreadable at the start of spreading during application, they tend to become less spreadable in the course of spreading. The present inventor has found through intensive study that the decreasing spreadability in the course of spreading is due to gelation of the cosmetic in the course of spreading, and that this gelation is surprisingly due to a spherical silicone powder included together therein, which has high affinity to silicone oil and high silicone oil absorption.
In view of the above-described circumstances, the present invention is directed to provide a cosmetic which provides easy-to-spread lightness at the start of application, in the course of application, and at the end of application, which stays smooth and soft when and after it is applied, and provides dry touch without oiliness during use.

Solution to Problem

An aspect of the cosmetic of the invention is a cosmetic comprising a silicone oil in an amount of 50 mass % or more relative to the total mass of the cosmetic and having a viscosity of 10000 mPa•s or less, the cosmetic comprising:

(a) a volatile linear silicone oil in an amount of 60 mass % or more relative to the total mass of the silicone oil; and
(b) an elastic powder having a linear silicone oil absorption of not less than 40 g/100 g and not more than 125 g/100 g and a rheometer hardness of not less than 40 and not more than 300 in an amount of 1 to 10 mass % relative to the total mass of the cosmetic, and
wherein the cosmetic contains an elastic powder having a linear silicone oil absorption higher than 125 g/100 g in an amount of not more than 3 mass % relative to the total mass of the cosmetic.

It is preferred that the elastic powder comprise diphenyldimethicone/vinyl diphenyldimethicone/silsesquioxane crosspolymer, polysilicone-22, or adipic acid/neopentyl glycol crosspolymer.

The cosmetic of the invention may be a water-in-oil type emulsion cosmetic.

Alternatively, the cosmetic of the invention may be an oil-based cosmetic.

Advantageous Effects of Invention

The cosmetic of the invention, which is a cosmetic comprising a silicone oil in an amount of 50 mass % or more relative to the total mass of the cosmetic and having a viscosity of 10000 mPa•s or less, and comprises: (a) a volatile linear silicone oil in an amount of 60 mass % or more relative to the total mass of the silicone oil; and (b) an elastic powder having a linear silicone oil absorption of not less than 40 g/100 g and not more than 125 g/100 g and a rheometer hardness of not less than 40 and not more than 300 in an amount of 1 to 10 mass % relative to the total mass of the cosmetic, and wherein the cosmetic contains an elastic powder having a linear silicone oil absorption higher than 125 g/100 g in an amount of not more than 3 mass % relative to the total mass of the cosmetic, can provide easy-to-spread lightness at the start of application, in the course of application, and at the end of application, can stay smooth and soft when and after the cosmetic is applied, and can provide dry touch without oiliness during use.

Description of Embodiments

Hereinafter, the cosmetic of the invention will be described in more detail.

The cosmetic of the invention is a cosmetic comprising a silicone oil in an amount of 50 mass % or more relative to the total mass of the cosmetic and having a viscosity of 10000 mPa•s or less, the cosmetic comprising: (a) a volatile linear silicone oil in an amount of 60 mass % or more relative to the total mass of the silicone oil; and (b) an elastic powder having a linear silicone oil absorption of not less than 40 g/100 g and not more than 125 g/100 g and a rheometer hardness of not less than 40 and not more than 300 in an amount of 1 to 10 mass % relative to the total mass of the cosmetic, and wherein the cosmetic contains an elastic powder having a linear silicone oil absorption higher than 125 g/100 g in an amount of not more than 3 mass % relative to the total mass of the cosmetic.

The individual components are described in detail below.

*(a) Volatile linear silicone oil*

The volatile linear silicone oil is a silicone oil having a boiling point of 250°C or less at 1 atom (101.325 kPa). Examples of the volatile linear silicone oil include low molecular weight methylpolysiloxanes (1cS, 1.5cS, 2cS, etc.), such as octamethyltrisiloxane, decamethyltrisiloxane, and dimethicone (dimethylpolysiloxane).

It is desirable that the content of the volatile linear silicone oil be 60 mass % or more, preferably 75 mass % or more, and desirably 80 mass % or more relative to the total mass of the silicone oil. The content of the volatile linear silicone oil of 60 mass % or more relative to the total mass of the silicone oil allows providing smooth skin when and after the cosmetic is applied, and providing dry touch without oiliness during use. The upper limit of the volatile linear silicone oil content is not particularly limited, and even the entire silicone oil may be the volatile linear silicone oil.

The content of the silicone oil is 50 mass % or more, and more preferably 55 mass % or more relative to the total mass of the cosmetic. The content of the volatile silicone oil of 50 mass % or more relative to the total mass of the cosmetic allows providing dry touch without oiliness during use. The upper limit of the silicone oil is not particularly limited; however, it is preferred that the content of the silicone oil be 90 mass % or less, and typically 80 mass % or less relative to the total mass of the cosmetic, since an excessively high content of the silicone oil results in largely decreased contents of the other components.

*Oily components other than the volatile linear silicone oil*

Examples of oily component other than the volatile linear silicone oil include a cyclic silicone oil, a non-volatile silicone oil, a polar oil component, a non-polar oil component, a solid oil component, a semi-solid oil component, an oil-soluble ultraviolet absorber, an oil-soluble agent, etc.

Examples of the cyclic silicone oil include cyclopentasiloxane, cyclohexanesiloxane, octamethylcyclotetrasiloxane, decamethylcyclopentasiloxane, dodecamethylcyclohexasiloxane, etc. Examples of the non-volatile silicone oil include high molecular weight dimethylpolysiloxane (for example, 6cS), etc.

Examples of the polar oil component include isopropyl myristate, octyldodecyl myristate, isopropyl palmitate, butyl stearate, hexyl laurate, myristyl myristate, decyl oleate, hexyldecyl dimethyloctanoate, cetyl lactate, myristyl lactate, lanolin acetate, isocetyl stearate, isocetyl isostearate, cholesteryl 12-hydroxystearate, ethylene glycol di-2-ethylhexanoate, dipentaerythritol fatty acid ester, N-alkyl glycol monoisostearate, neopentyl glycol dicaprate, diisostearyl malate, glycerin di-2-heptylundecanoate, trimethylolpropane tri-2-ethylhexanoate, pentaneerythritol tetra-2-ethylhexanoate, glycerin tri-2-ethylhexanoate, trimethylolpropane triisostearate, cetyl 2-ethylhexanoate, 2-ethylhexyl palmitate, glycerin trimyristate, glyceryl tri(caprylate/caprate), triethylhexanoin, cetyl ethylhexanoate, polyglyceryl-2 triisostearate, dipentaerythrityl hexahydroxystearate, pentaerythrityl tetra(behenate/benzoate/ethylhexanoate), PPG-3 dipivalate, dipentaerythrityl tripolyhydroxystearate, pentaerythrityl tetra(ethylhexanoate/benzoate), Macadamia seed oil polyglyceryl-6 esters behenate, (phytosteryl/behenyl) dimer dilinoleate, lanolin, diethylhexyl succinate, lanolin fatty acid octyldodecyl, isostearyl palmitate, diheptylundecyl adipate, isocetyl myristate, dihexyl decyl adipate, diisopropyl sebacate, pentaerythrityl tetraethylhexanoate, glyceride tri-2-heptylundecanoate, castor oil fatty acid methylester, oleyl oleate, cetostearyl alcohol, acetoglyceride, 2-heptylundecyl palmitate, diisobutyl adipate, N-lauroyl-L-glutamate-2-octyldodecyl, di-2-heptylundecyl adipate, ethyllaurate, di-2-ethylhexyl sebacate, 2-hexyl decyl myristate, 2-hexyl decyl palmitate, 2-hexyl decyl adipate, diisopropyl sebacate, 2-ethylhexyl succinate, ethyl acetate, butyl acetate, amyl acetate, triethyl citrate, triglycerin, glycerin trioctanoate, and glycerin triisopalmitate.

Examples of the non-polar oil component include hydrocarbon oils, such as liquid paraffin, squalane, squalene, paraffin, isododecane, isohexadecane, and hydrogenerated polydecene.

Examples of the solid oil component include: solid oils, such as cacao butter, coconut oil, horse oil, hydrogenated palm oil, palm oil, beef tallow, mutton tallow, and hydrogenated castor oil; hydrocarbons, such as paraffin wax (linear hydrocarbon), microcrystalline wax (branched saturated hydrocarbon), ceresin wax, Japan wax, montan wax, and Fischer-Tropsch wax; waxes, such as bees wax, lanolin, carnauba wax, candelilla wax, rice bran wax (rice wax), spermaceti wax, jojoba oil, bran wax, montan wax, kapok wax, bay berry wax, shellac wax, sugar cane wax, lanolin fatty acid isopropyl, hexyl laurate, reduced lanolin, hard lanolin, POE (polyoxyethylene) lanolin alcohol ether, POE lanolin alcohol acetate, POE cholesterol ether, lanolin fatty acid polyethylene glycol, and POE hydrogenated lanolin alcohol ether; higher fatty acids, such as myristic acid, palmitic acid, stearic acid, and behenic acid; and higher alcohols, such as cetyl alcohol, stearyl alcohol, behenyl alcohol, myristyl alcohol, and cetostearyl alcohol, etc.

Examples of the semi-solid oil component include Vaseline, lanolin, vegetable fats, such as shea butter, and partially hydrogenerated palm oil, partially hydrogenerated jojoba oil, bis-diglyceryl polyacyladipate-2, pentaerythrityl tetra(behenate/benzoate/ethylhexanoate), Macadamia seed oil polyglyceryl-6 esters behenate, (phytosteryl/behenyl) dimer dilinoleate, dipentaerithrite hexaoxystearate, etc.

Examples of the oil-soluble ultraviolet absorber include: cinnamate ultraviolet absorbers, such as octyl para-methoxy cinnamate (ethylhexyl methoxycinnamate), isopropyl para-methoxy cinnamate, and mono-2-ethylhexanoate glyceryl di-para-methoxy cinnamate; benzoate ultraviolet absorbers, such as para-amino benzoate; anthranilate ultraviolet absorbers, such as methyl anthranilate; salicylate ultraviolet absorbers, such as octyl salicylate, and phenyl salicylate; 4-tert-butyl-4'-methoxy benzoyl methane, 2-ethylhexyl 2-cyano-3,3-diphenyl acrylate, etc.

Examples of the oil-soluble agent include: oil-soluble vitamins, such as vitamin A (retinol), vitamin D, vitamin E, vitamin K, and derivatives thereof (vitamin A oil, retinol palmitate, etc.); oil-soluble derivatives of water-soluble chemicals, such as vitamin C and arbutin (vitamin C palmitate, etc.); oil-soluble botanical extract; oil-soluble fragrance; surface-hydrophobized materials; cyclosporine, etc.

*(b) Elastic powder*

The elastic powder has a linear silicone oil absorption of not less than 40 g/100 g and not more than 125 g/100 g, and a rheometer hardness of not less than 40 and not more than 300.

The linear silicone oil is a dimethyl silicone (silicone KF-96A-6T, available from Shin-Etsu Chemical Co., Ltd.) The oil absorption is not less than 40 g/100 g and not more than 125 g/100 g, preferably not less than 50 g/100 g and not more than 110 g/100 g, and desirably not less than 55 g/100 g and not more than 100 g/100 g. The oil absorption not more than 125 g/100 g allows limiting the affinity to the volatile linear silicone oil, thereby providing easy-to-spread lightness in the course of application as well as at the start of application of the cosmetic. The oil absorption not less than 40 g/100 g allows successfully maintaining smoothness during application.

The rheometer hardness of the elastic powder is not less than 40 and not more than 300, preferably not less than 50 and not more than 250, and desirably not less than 60 and not more than 220. This rheometer hardness is a value measured using SUN rheometer (CMPAC-II, available from SUN SCIENTIFIC CO., LTD.), where 2 g of the elastic powder is filled in a container having a diameter of 30 mm and the surface of the powder sample is flattened, a disk probe having a diameter of 30 mm is penetrated into the sample to a depth of 2 mm at a rate of 20 mm/min, and then the range value is read. The rheometer hardness of 300 or less of the elastic powder allows providing softness in the course of application as well as at the start of application of the cosmetic. The rheometer hardness of 40 or more of the elastic powder allows providing the cosmetic that is less likely to form uneven spreading on the skin and can be smoothly spread.

The elastic powder is preferably a silicone composite powder formed by coating spherical silicone rubber particles with a silicone resin. Preferred examples thereof include diphenyldimethicone/vinyl diphenyldimethicone/silsesquioxane crosspolymer, polysilicone-22, etc., and preferred examples of commercially available products thereof include KSP-300 and KSP-441 (available from Shin-Etsu Chemical Co., Ltd.) Besides the silicone composite powder, adipic acid/neopentyl glycol crosspolymer may be used, and an example of commercially available product thereof is Penstia™ Powder (available from Center Chem). Also, nylon-12 may be used, and a preferred example of commercially available product thereof is NYLON SP-500.

The elastic powder may be used alone or in combination of two or more, as appropriate.

The content of the elastic powder is 1 to 10 mass %, preferably 1.5 to 7.5 mass %, and desirably 2 to 7 mass % relative to the total mass of the cosmetic. The content of the elastic powder of 1 to 10 mass % relative to the total mass of the cosmetic allows providing the cosmetic that provides easy-to-spread lightness at the start of application, in the course of application, and at the end of application, stays smooth and soft when and after it is applied, and provides dry touch without oiliness during use.

The content of the elastic powder having a linear silicone oil absorption higher than 125 g/100 g is not more than 3 mass % relative to the total mass of the cosmetic. If the content of the elastic powder having a linear silicone oil absorption higher than 125 g/100 g exceeds 3 mass %, spreadability of the cosmetic decreases in the course of application, resulting in heaviness just before the finger motion was stopped at the end of application. It is preferred that the contend of elastic powder having a linear silicone oil absorption less than 40 g/100 g be 1 mass % or less, in view of maintaining smoothness during application, although it depends on the contents of the other components.

The cosmetic of the invention has a viscosity of 10000 mPa•s or less, preferably 500 to 8500 mPa•s, and desirably 1500 to 7500 mPa•s. This viscosity is a value measured under measurement conditions: 30°C, a BL-type viscometer with a #3 rotor, 12 rpm, and 1 minute. The viscosity of 10000 mPa•s or less of the cosmetic allows providing easy-to-spread lightness at the start of application, in the course of application, and at the end of application.

*Inorganic powders*

The cosmetic of the invention may contain inorganic powders that are usually allowed to be contained in cosmetics.

Specific preferred examples thereof include titanium oxide, zinc oxide, black iron oxide, yellow iron oxide, colcothar, ultramarine, iron blue, talc, mica, sericite, kaolin, titanium dioxide-coated mica, chromium oxide, and chromium hydroxide.

Such an inorganic powder may be used without any treatment; however, it is preferred to use a hydrophobized inorganic powder prepared by hydrophobizing the surfaces of the inorganic powder.

The hydrophobized inorganic powder is prepared by hydrophobizing an inorganic powder with a silicone, such as methyl hydrogen polysiloxane or dimethylpolysiloxane, or a hydrocarbon, such as a dextrin fatty acid ester, a higher fatty acid, a higher alcohol, a fatty acid ester, a metal soap, an alkyl ether phosphate, a fluorine compound, squalane, or paraffin, using a wet process using a solvent, a vapor process, a mechanochemical process, or the like.

The average particle size of the powders is not particularly limited; however, powders having an average primary particle size in the range from 0.001 $\mu$m to 100 $\mu$m, and preferably 0.001 $\mu$m to 10 $\mu$m are used. It is preferred that the average particle size be adjusted to be smaller than emulsion particles of the oil phase. It should be noted that the term "fine particulate" as used herein refers to powder having an average primary particle size in the range from 0.001 to 0.1 $\mu$m, and the term "pigment-grade" as used herein refers to powder having an average primary particle size in the range from 0.2 to 0.4 $\mu$m.

It is preferred that 2/3 or more of the content of the powders contained in the cosmetic of the invention is occupied by powders having been subjected to a certain hydrophobization, in view of improving stability.

The content of the inorganic powders is preferably 0.1 mass % or more, and more preferably 5 mass % or more relative to the total mass of the cosmetic. If the content of the inorganic powders is excessively small, the sedimentation velocity of the powders may become high. The content of the inorganic powders is preferably 50 mass % or less, more preferably 30 mass % or less, and particularly preferably 20 mass % or less. If the content of the inorganic powders is excessively large, the viscosity may become high.

The cosmetic of the invention may have any form, and may be provided in any form, such as an emulsified system, or a water-oil double layer system.

The cosmetic of the invention can be used as produced, can be diluted with an oily component to form an oil-based cosmetic, or can be emulsified with an aqueous component according to a known method to form a water-in-oil type emulsion cosmetic.

In the case where an aqueous component is used, one that is usually allowed to be contained in cosmetics can be used within a range where the stability of the cosmetic is not impaired.

Examples of such an aqueous component include water, a lower alcohol, a moisturizing agent, a water-soluble thickener, a water-soluble ultraviolet absorber, a sequestering agent, an antioxidant, and a water-soluble chemical.

Further, the cosmetic of the invention can contain other components (such as an anionic surfactant, a cationic surfactant, an amphoteric surfactant, a dispersant, a thickener, a film former, a saccharide, an amino acid, an organic amine, a polymeric emulsion, a pH adjuster, a skin nutrient, a vitamin, an antioxidant aid, a fragrance, etc.) that are usually allowed to be contained in cosmetics in a range where the advantageous effects of the invention are not impaired.

The cosmetic of the invention is applicable to water-in-oil type emulsion cosmetics, and oil-based cosmetics. Examples of such cosmetics include facial liquid foundation, makeup base, and sunscreen.

Examples

Now, the present invention is described in more detail with reference to examples, which are not intended to limit the invention. In the following examples, all compositions are given in mass percent unless otherwise noted.

Cosmetics having compositions shown in Tables 1 and 2 below were prepared according to common procedures. The viscosity of each cosmetic and the oil absorption and the hardness of each powder used in the examples and comparative examples were measured as described below.

*Viscosity*

The viscosity of each of the resulting cosmetics was measured under measurement conditions: 30°C, a BL-type viscometer (type VS-A, available from Shibaura System) with a #3 rotor, 12 rpm, and 1 minute.

*Oil absorption*

The oil absorption was measured according to JIS K-5101. Namely, 1g of each powder was precisely measured and put on a glass plate. Then, silicone KF-96A-6T was dripped on the center of the powder little by little, and the powder and the silicone were evenly kneaded with a spatula every time the silicone was dripped until the entire mixture became a paste, with the end point being just before the mixture became a fluid. Then, the oil absorption was calculated according to the formula below:

$$\text{Oil absorption (g/100 g)} = (\text{silicone KF-96A-6T (g)} / \text{amount of sample (g)}) \times 100.$$

*Hardness*

The hardness was measured using SUN rheometer (CMPAC-II, available from SUN SCIENTIFIC CO., LTD.), where 2 g of the elastic powder was filled in a container having a diameter of 30 mm and the surface of the powder sample was flattened, a disk probe having a diameter of 30 mm was penetrated into the sample to a depth of 2 mm at a rate of 20 mm/min, and then the range value was read.

The oil absorption and the hardness measured for each powder used in the examples were as shown below.
Adipic acid/neopentyl glycol crosspolymer: oil absorption 73 g/100 g, hardness 73.0
Vinyl dimethicone/methicone silsesquioxane crosspolymer: oil absorption 150 g/100 g, hardness 121.0
Diphenyldimethicone/vinyl diphenyldimethicone/silsesquioxane crosspolymer: oil absorption 45 g/100 g, hardness 218.0
Polysilicone-22: oil absorption 100 g/100 g, hardness 66.0
dimethicone/vinyl dimethicone crosspolymer: oil absorption 400 g/100 g, hardness 34.0
Nylon -12: oil absorption 67 g/100 g, hardness 90.0
Methyl methacrylate crosspolymer: oil absorption 50 g/100 g, hardness 2000 or more
Silica: oil absorption 60 g/100 g, hardness 2000 or more
Calcium carbonate: oil absorption 45 g/100 g, hardness 2000 or more
Polyethylene (95%) silica (5%) mixture: oil absorption 60 g/100 g, hardness 363.0

Feel during use of the resulting cosmetics were evaluated according to the following criteria.

*Easy-to-spread lightness at the start of application*

A panel of ten female specialists actually applied each cosmetic to their skin, and evaluated easy-to-spread lightness at the start of application.

A: Eight or more out of the panel felt easy-to-spread lightness at the start of application.
B: Six to seven out of the panel felt easy-to-spread lightness at the start of application.
C: Three to five out of the panel felt easy-to-spread lightness at the start of application.
D: Two or less out of the panel felt easy-to-spread lightness at the start of application.

*Lightness in the course of application*

The panel of ten female specialists actually applied each cosmetic to their skin, and evaluated lightness in the course of application.

A: Eight or more out of the panel felt lightness in the course of application.
B: Six to seven out of the panel felt lightness in the course of application.
C: Three to five out of the panel felt lightness in the course of application.
D: Two or less out of the panel felt lightness in the course of application.

*Lightness at the end of application*

The panel of ten female specialists actually applied each cosmetic to their skin, and evaluated lightness just before the finger motion was stopped at the end of application.

A: Eight or more out of the panel felt lightness just before the finger motion was stopped at the end of application.
B: Six to seven out of the panel felt lightness just before the finger motion was stopped at the end of application.
C: Three to five out of the panel felt lightness just before the finger motion was stopped at the end of application.
D: Two or less out of the panel felt lightness just before the finger motion was stopped at the end of application.

*Smoothness*

The panel of ten female specialists actually applied each cosmetic to their skin, and evaluated feel during use (smoothness) during application.

A: Eight or more out of the panel felt smoothness during application.
B: Six to seven out of the panel felt smoothness during application.
C: Three to five out of the panel felt smoothness during application.
D: Two or less out of the panel felt smoothness during application.

*Softness*

The panel of ten female specialists actually applied each cosmetic to their skin, and evaluated feel during use (softness) during application.

A: Eight or more out of the panel felt softness during application.
B: Six to seven out of the panel felt softness during application.
C: Three to five out of the panel felt softness during application.
D: Two or less out of the panel felt softness during application.

*Oiliness*

The panel of ten female specialists actually applied each cosmetic to their skin, and evaluated oiliness during application.

A: Eight or more out of the panel felt no oiliness during application.
B: Six to seven out of the panel felt no oiliness during application.
C: Three to five out of the panel felt no oiliness during application.
D: Two or less out of the panel felt no oiliness during application.

The results of the evaluation are shown in Tables 1 and 2 together with the compositions. The product names of the ingredients shown in Tables 1 and 2 are as shown below.
Dimethicone (tetramer): KF-96L-1.5cS
Dimethicone (mixture of 3:1 trimer and tetramer): BELSIL® DM1 PLUS
Adipic acid/neopentyl glycol crosspolymer: Penstia™ Powder
Vinyl dimethicone/methicone silsesquioxane crosspolymer: silicone powder KSP100
Diphenyldimethicone/vinyl diphenyldimethicone/silsesquioxane crosspolymer: silicone powder KSP300
Polysilicone-22: silicone powder KSP441

Dimethicone/vinyl dimethicone crosspolymer: TREFIL E506W
Nylon -12: Nylon SP-500
Polyethylene (95%) silica (5%) mixture: spherical polyethylene SS-1
Methyl methacrylate crosspolymer: MICROSPHERE M306
Silica: SATINIER M5
Calcium carbonate: CALMARU SCS-M5
Disteardimonium hectorite: BENTON 38VCG

[Table 1]

| | Ingredients | Example 1 | Example 2 | Example 3 | Example 4 | Example 5 | Example 6 | Comp. Ex. 1 | Comp. Ex. 2 | Comp. Ex. 3 | Comp. Ex. 4 | Comp. Ex. 5 | Comp. Ex. 6 | Comp. Ex. 7 | Comp. Ex. 8 |
|---|---|---|---|---|---|---|---|---|---|---|---|---|---|---|---|
| Oil phase | Dimethicone (tetramer) | 30 | 30 | 29.25 | 28.5 | 30 | 30 | 30 | 28 | 27.5 | 30 | 30 | 30 | 30 | 30 |
| | Dimethicone (mixture of 3:1 trimer and tetramer) | 30 | 30 | 29.25 | 28.5 | 30 | 30 | 30 | 28 | 27.5 | 30 | 30 | 30 | 30 | 30 |
| | Decamethylcyclopentasiloxane | | | | | | | | | | | | | | |
| | Glyceryl tri (caprylate/caprate) | 3 | 3 | 3 | 3 | 3 | 3 | 3 | 3 | 3 | 3 | 3 | 3 | 3 | 3 |
| | Ethylhexyl methoxycinnamate | 7 | 7 | 7 | 7 | 7 | 7 | 7 | 7 | 7 | 7 | 7 | 7 | 7 | 7 |
| Surfactant | PEG-9 polydimethylsiloxvethyl dimethicone | 1 | 1 | 1 | 1 | 1 | 1 | 1 | 1 | 1 | 1 | 1 | 1 | 1 | 1 |
| Dispersant | Isostearic acid | 0.5 | 0.5 | 0.5 | 0.5 | 0.5 | 0.5 | 0.5 | 0.5 | 0.5 | 0.5 | 0.5 | 0.5 | 0.5 | 0.5 |
| Powder | Adipic acid/ neopentyl glycol crosspolymer | 5 | | | | | | | | | | | | | |

| Ingredients | Example 1 | Example 2 | Example 3 | Example 4 | Example 5 | Example 6 | Comp. Ex. 1 | Comp. Ex. 2 | Comp. Ex. 3 | Comp. Ex. 4 | Comp. Ex. 5 | Comp. Ex. 6 | Comp. Ex. 7 | Comp. Ex. 8 |
|---|---|---|---|---|---|---|---|---|---|---|---|---|---|---|
| Vinyl dimethicone/methicone silsesquioxane crosspolymer | | | 1.5 | 3 | | | 5 | 4 | 5 | | | | | |
| Diphenyldimethicone/ vinyl diphenyldimethicone/ silsesquioxane crosspolymer | | 5 | 5 | 5 | | | | 5 | 5 | | | | | |
| Polysilicone-22 | | | | | 5 | | | | | | | | | |
| Dimethicone/vinyl dimethicone crosspolymer | | | | | | | | | | 5 | | | | |
| Nylon -12 | | | | | | 5 | | | | | | | | |
| Polyethylene (95%) silica (5%) mixture | | | | | | | | | | | | | 5 | |
| Methyl methacrylate crosspolymer | | | | | | | | | | | 5 | | | |
| Silica | | | | | | | | | | | | 5 | | |

EP 3 357 488 B1

| | Ingredients | Example 1 | Example 2 | Example 3 | Example 4 | Example 5 | Example 6 | Comp. Ex. 1 | Comp. Ex. 2 | Comp. Ex. 3 | Comp. Ex. 4 | Comp. Ex. 5 | Comp. Ex. 6 | Comp. Ex. 7 | Comp. Ex. 8 |
|---|---|---|---|---|---|---|---|---|---|---|---|---|---|---|---|
| | Calcium carbonate | | | | | | | | | | | | | 5 | |
| Thickener | Disteardimonium hectorite | 1.6 | 1.6 | 1.6 | 1.6 | 1.6 | 1.6 | 1.6 | 1.6 | 1.6 | 1.6 | 1.6 | 1.6 | 1.6 | 1.6 |
| Inorganic Powder | Hydrophobized particulate titanium oxide (metal soap-treated) | 3 | 3 | 3 | 3 | 3 | 3 | 3 | 3 | 3 | 3 | 3 | 3 | 3 | 3 |
| | Hydrophobized pigment-grade titanium oxide (silicone-treated) | 6 | 6 | 6 | 6 | 6 | 6 | 6 | 6 | 6 | 6 | 6 | 6 | 6 | 6 |
| | Hydrophobized yellow iron oxide (silicone-treated) | 1.2 | 1.2 | 1.2 | 1.2 | 1.2 | 1.2 | 1.2 | 1.2 | 1.2 | 1.2 | 1.2 | 1.2 | 1.2 | 1.2 |
| | Hydrophobized red iron oxide (silicone-treated) | 0.4 | 0.4 | 0.4 | 0.4 | 0.4 | 0.4 | 0.4 | 0.4 | 0.4 | 0.4 | 0.4 | 0.4 | 0.4 | 0.4 |
| | Hydrophobized black iron oxide (silicone-treated) | 0.05 | 0.05 | 0.05 | 0.05 | 0.05 | 0.05 | 0.05 | 0.05 | 0.05 | 0.05 | 0.05 | 0.05 | 0.05 | 0.05 |

EP 3 357 488 B1

(continued)

| Ingredients | | Example 1 | Example 2 | Example 3 | Example 4 | Example 5 | Example 6 | Comp. Ex. 1 | Comp. Ex. 2 | Comp. Ex. 3 | Comp. Ex. 4 | Comp. Ex. 5 | Comp. Ex. 6 | Comp. Ex. 7 | Comp. Ex. 8 |
|---|---|---|---|---|---|---|---|---|---|---|---|---|---|---|---|
| Aqueous phase | Ion-exchanged water | 9.25 | 9.25 | 9.25 | 9.25 | 9.25 | 9.25 | 9.25 | 9.25 | 9.25 | 9.25 | 9.25 | 9.25 | 9.25 | 9.25 |
| | 95% alcohol | 2 | 2 | 2 | 2 | 2 | 2 | 2 | 2 | 2 | 2 | 2 | 2 | 2 | 2 |
| Total | | 100 | 100 | 100 | 100 | 100 | 100 | 100 | 100 | 100 | 100 | 100 | 100 | 100 | 100 |
| (Silicone oil / total cosmetic material) × 100 | | 60 | 60 | 58.5 | 57 | 60 | 60 | 60 | 56 | 55 | 60 | 60 | 60 | 60 | 60 |
| (Volatile linear silicone oil / total silicone oil) × 100 | | 100 | 100 | 100 | 100 | 100 | 100 | 100 | 100 | 100 | 100 | 100 | 100 | 100 | 100 |
| Viscosity | | 2270 | 2040 | 2200 | 2890 | 2400 | 1500 | 2340 | 3500 | 3910 | 3340 | 1200 | 2050 | 870 | 1180 |
| Easy-to-spread lightness at the start of application | | A | A | A | A | A | A | A | B | B | B | A | A | A | A |
| Lightness in the course of application | | A | A | A | B | A | A | B | B | B | B | A | A | A | B |
| Lightness at the end of application | | A | B | B | B | A | A | C | C | C | D | A | A | A | C |
| Smoothness | | A | B | B | B | A | B | B | B | B | A | C | C | D | B |
| Softness | | A | B | B | B | A | B | B | B | B | A | C | C | D | B |
| Non-oiliness | | A | A | A | A | A | A | A | A | A | A | A | A | A | A |

[Table 2]

| | Ingredients | Example 7 | Comp. Ex. 9 | Comp. Ex. 10 | Example 8 | Comp. Ex. 11 | Comp. Ex. 12 | Example 9 | Comp. Ex. 13 | Comp. Ex. 14 | Example 10 | Example 11 | Example 12 |
|---|---|---|---|---|---|---|---|---|---|---|---|---|---|
| Oil phase | Dimethicone (tetramer) | 25 | 20 | 22.5 | 18 | | 9 | 25 | 22.5 | 20 | 25 | 18 | 25 |
| | Dimethicone (mixture of 3:1 trimer and tetramer) | 25 | 20 | 22.5 | 18 | | 9 | 25 | 22.5 | 20 | 25 | 18 | 25 |
| | Decamethylcyclo pentasiloxane | | | | 24 | 60 | 42 | | | | | 24 | |
| | Glyceryl tri (caprylate/ caprate) | 13 | 23 | 18 | 3 | 3 | 3 | | | | 13 | 3 | |
| | Ethylhexyl methoxycinnamate | 7 | 7 | 7 | 7 | 7 | 7 | 7 | 7 | 7 | 7 | 7 | 7 |
| Surfactant | PEG-9 polydimethylsiloxyethyl dimethicone | 1 | 1 | 1 | 1 | 1 | 1 | 1 | 1 | 1 | 1 | 1 | 1 |
| Dispersant | Isostearic acid | 0.5 | 0.5 | 0.5 | 0.5 | 0.5 | 0.5 | 0.5 | 0.5 | 0.5 | 0.5 | 0.5 | 0.5 |

EP 3 357 488 B1

13

(continued)

| | Ingredients | Example 7 | Comp. Ex. 9 | Comp. Ex. 10 | Example 8 | Comp. Ex. 11 | Comp. Ex. 12 | Example 9 | Comp. Ex. 13 | Comp. Ex. 14 | Example 10 | Example 11 | Example 12 |
|---|---|---|---|---|---|---|---|---|---|---|---|---|---|
| Powder | Adipic acid/ neopentyl glycol crosspolymer | | | | | | | | | | 5 | 5 | 5 |
| | Vinyl dimethicone/ methicone silsesquioxane crosspolymer | | | | | | | | | | | | |
| | Diphenyldimethicone/vinyl diphenyldimethicone/ silsesquioxane crosspolymer | | | | | | | | | | | | |
| | Polysilicone-22 | 5 | 5 | 5 | 5 | 5 | 5 | 5 | 5 | 5 | | | |
| | Dimethicone/ vinyl dimethicone crosspolymer | | | | | | | | | | | | |
| | Nylon -12 | | | | | | | | | | | | |
| | Polyethylene (95%) silica (5%) mixture | | | | | | | | | | | | |
| | Methyl methacrylate crosspolymer | | | | | | | | | | | | |
| | Silica | | | | | | | | | | | | |
| | Calcium carbonate | | | | | | | | | | | | |
| Thickener | Disteardimonium hectorite | 1.6 | 1.6 | 1.6 | 1.6 | 1.6 | 1.6 | 1.6 | 1.6 | 1.6 | 1.6 | 1.6 | 1.6 |

(continued)

| | Ingredients | Example 7 | Comp. Ex. 9 | Comp. Ex. 10 | Example 8 | Comp. Ex. 11 | Comp. Ex. 12 | Example 9 | Comp. Ex. 13 | Comp. Ex. 14 | Example 10 | Example 11 | Example 12 |
|---|---|---|---|---|---|---|---|---|---|---|---|---|---|
| Inorganic Powder | Hydrophobized particulate titanium oxide (metal soap-treated) | 3 | 3 | 3 | 3 | 3 | 3 | 3 | 3 | 3 | 3 | 3 | 3 |
| | Hydrophobized pigment-grade titanium oxide (silicone-treated) | 6 | 6 | 6 | 6 | 6 | 6 | 6 | 6 | 6 | 6 | 6 | 6 |
| | Hydrophobized yellow iron oxide (silicone-treated) | 1.2 | 1.2 | 1.2 | 1.2 | 1.2 | 1.2 | 1.2 | 1.2 | 1.2 | 1.2 | 1.2 | 1.2 |
| | Hydrophobized red iron oxide (silicone-treated) | 0.4 | 0.4 | 0.4 | 0.4 | 0.4 | 0.4 | 0.4 | 0.4 | 0.4 | 0.4 | 0.4 | 0.4 |
| | Hydrophobized black iron oxide (silicone-treated) | 0.05 | 0.05 | 0.05 | 0.05 | 0.05 | 0.05 | 0.05 | 0.05 | 0.05 | 0.05 | 0.05 | 0.05 |
| Aqueous phase | Ion-exchanged water | 9.25 | 9.25 | 9.25 | 9.25 | 9.25 | 9.25 | 22.25 | 27.25 | 32.25 | 9.25 | 9.25 | 22.25 |
| | 95% alcohol | 2 | 2 | 2 | 2 | 2 | 2 | 2 | 2 | 2 | 2 | 2 | 2 |
| Total | | 100 | 100 | 100 | 100 | 100 | 100 | 100 | 100 | 100 | 100 | 100 | 100 |
| (Silicone oil / total cosmetic material) $\times$ 100 | | 50 | 40 | 45 | 60 | 60 | 60 | 50 | 45 | 40 | 50 | 60 | 50 |
| (Volatile linear silicone oil / total silicone oil) $\times$ 100 | | 100 | 100 | 100 | 60 | 0 | 30 | 100 | 100 | 100 | 100 | 60 | 100 |
| Viscosity | | 7300 | 6860 | 5000 | 3000 | 3700 | 4600 | 3500 | 6500 | 8340 | 7030 | 2800 | 3220 |
| Easy-to-spread lightness at the start of application | | B | B | B | A | B | B | B | B | C | B | A | B |

EP 3 357 488 B1

(continued)

| | Ingredients | Example 7 | Comp. Ex. 9 | Comp. Ex. 10 | Example 8 | Comp. Ex. 11 | Comp. Ex. 12 | Example 9 | Comp. Ex. 13 | Comp. Ex. 14 | Example 10 | Example 11 | Example 12 |
|---|---|---|---|---|---|---|---|---|---|---|---|---|---|
| Lightness in the course of application | | B | B | A | A | A | A | B | C | C | B | A | B |
| Lightness at the end of application | | B | B | A | A | A | A | B | C | C | B | A | B |
| Smoothness | | A | A | A | B | C | C | A | A | A | A | B | A |
| Softness | | A | A | A | A | A | A | A | A | A | A | A | A |
| Non-oiliness | | B | D | C | A | C | B | A | A | A | B | A | A |

As can clearly be seen from Table 1, each of the cosmetics of Examples 1 to 6 provided easy-to-spread lightness at the start of application, in the course of application, and at the end of application, stayed smooth and soft when and after the cosmetic was applied, and provided dry touch without oiliness during use. In contrast, the cosmetics of Comparative Examples 1, and 4 to 8, which used powders that did not meet the requirements of oil absorption and rheometer hardness specified in the invention, provided heavy spreadability in the course of application and at the end of application, and did not provide a satisfactory level of feel during use with respect to spreadability, smoothness, and softness. The cosmetics of Comparative Examples 2 and 3, which used powders that met the requirements of oil absorption and rheometer hardness specified in the invention, where the content of the elastic powder having an oil absorption higher than 125 g/100 g was more than 3 mass % relative to the total mass of the cosmetic, exhibited poor spreadability at the end of application and heaviness at the end of application, as can clearly be seen from comparison with Examples 3 and 4.

Further, as can be seen from Table 2, comparing Example 7 with Comparative Examples 9 and 10, the cosmetics of Comparative Examples 9 and 10, where the silicone oil content was not 50 mass % or more relative to the total mass of the cosmetic, did not provide dry touch without oiliness during use. Comparing Example 8 with Comparative Examples 11 and 12, the cosmetics of Comparative Examples 11 and 12 where the content of the volatile linear silicone oil was not 60 mass % or more relative to the total mass of the silicone oil, did not provide a satisfactory level of smoothness and non-oiliness. Comparing Example 9 with Comparative Examples 13 and 14, the cosmetics of Comparative Examples 13 and 14, where the content of the silicone oil was not 50 mass % or more relative to the total mass of the cosmetic and contained rich water, did not provide sufficient lightness in the course of and at the end of application. In addition, as can be seen from Examples 10, 11, and 12, each cosmetic which contains the elastic powder having a linear silicone oil absorption of not less than 40 g/100 g and not more than 125 g/100 g and a rheometer hardness of not less than 40 and not more than 300, exhibited similar effects.

*Example Formulations*

Example formulations of the cosmetic of the invention are shown below. These example formulations are not intended to limit the invention. All compositions are given in mass percent relative to the total amount of each product.

*Example Formulation 1: facial liquid foundation (water-in-oil type)*

Volatile linear silicone (1.5cS): 65%
Ethylhexyl methoxycinnamate: 5%
Dimethicone crosspolymer (Dow Corning 9045 Silicone Elastomer Blend): 1%
PEG-9 polydimethylsiloxyethyl dimethicone: 2%
Isostearic acid: 1%
Polysilicone-22: 3%
Diphenyldimethicone/vinyl diphenyldimethicone/silsesquioxane crosspolymer: 2%
Hydrophobized fine particulate titanium oxide: 5%
Hydrophobized pigment-grade titanium oxide: 6%
Hydrophobized yellow iron oxide: 1.2%
Hydrophobized yellow iron oxide: 0.4%
Hydrophobized black iron oxide: 0.05%
Antiseptic: appropriate amount
Ion-exchanged water: balance

*Example Formulation 2: facial liquid foundation (water-in-oil type)*

Volatile linear silicone (1.5cS): 65%
Ethylhexyl methoxycinnamate: 5%
Acrylates/polytrimethylsiloxymethacrylate copolymer: 3%
PEG-9 polydimethylsiloxyethyl dimethicone: 2%
Isostearic acid: 1%
Polysilicone-22: 3%
Diphenyldimethicone/vinyl diphenyldimethicone/silsesquioxane crosspolymer: 2%
Hydrophobized fine particulate titanium oxide: 5%
Hydrophobized pigment-grade titanium oxide: 6%
Hydrophobized yellow iron oxide: 1.2%
Hydrophobized yellow iron oxide: 0.4%
Hydrophobized black iron oxide: 0.05%
Antiseptic: appropriate amount
Ion-exchanged water: balance

*Example Formulation 3: facial liquid foundation (water-in-oil type)*

Volatile linear silicone (1.5cS): 65%
Ethylhexyl methoxycinnamate: 5%
Trifluoroalkyl dimethyl ·trimethylsiloxysilicate: 5%
PEG-9 polydimethylsiloxyethyl dimethicone: 2%
Isostearic acid: 1%
Polysilicone-22: 3%
Diphenyldimethicone/vinyl diphenyldimethicone/silsesquioxane crosspolymer: 2%
Hydrophobized fine particulate titanium oxide: 5%
Hydrophobized pigment-grade titanium oxide: 6%
Hydrophobized yellow iron oxide: 1.2%
Hydrophobized yellow iron oxide: 0.4%
Hydrophobized black iron oxide: 0.05%
Antiseptic: appropriate amount
Ion-exchanged water: balance

*Example Formulation 4: facial liquid foundation (oil-based)*

Volatile linear silicone (1.5cS): balance
Ethylhexyl methoxycinnamate: 7.5%
Acrylates/polytrimethylsiloxymethacrylate copolymer: 3%
PEG-9 polydimethylsiloxyethyl dimethicone: 1.5%
Isostearic acid: 0.5%
Polysilicone-22: 2.5%
Diphenyldimethicone/vinyl diphenyldimethicone/silsesquioxane crosspolymer: 2.5%
Hydrophobized fine particulate titanium oxide: 5%
Hydrophobized pigment-grade titanium oxide: 6%
Hydrophobized yellow iron oxide: 1.2%
Hydrophobized yellow iron oxide: 0.4%
Hydrophobized black iron oxide: 0.05%

*Example Formulation 5: facial liquid foundation (oil-based)*

Volatile linear silicone (1.5cS): balance
Ethylhexyl methoxycinnamate: 7.5%
Trifluoroalkyl dimethyl ·trimethylsiloxysilicate: 5%
PEG-9 polydimethylsiloxyethyl dimethicone: 1.5%
Isostearic acid: 0.5%
Polysilicone-22: 2.5%
Diphenyldimethicone/vinyl diphenyldimethicone/silsesquioxane crosspolymer: 2.5%
Hydrophobized fine particulate titanium oxide: 5%
Hydrophobized pigment-grade titanium oxide: 6%
Hydrophobized yellow iron oxide: 1.2%
Hydrophobized yellow iron oxide: 0.4%
Hydrophobized black iron oxide: 0.05%

*Example Formulation 6: sunscreen (water-in-oil type)*

Volatile linear silicone (1.5cS): 50%
Dodecamethylcyclohexasiloxane: 5%
Ethylhexyl methoxycinnamate: 7.5%
Disteardimonium hectorite: 1%
PEG-10 dimethicone: 1.5%
Isostearic acid: 1%
Adipic acid/neopentyl glycol crosspolymer: 3%
Hydrophobized fine particulate zinc oxide: 5%
Hydrophobized fine particulate titanium oxide: 5%

Antiseptic: appropriate amount
Ion-exchanged water: balance

*Example Formulation 7: sunscreen (oil-based)*

Volatile linear silicone (1.5cS): balance
Dodecamethylcyclohexasiloxane: 5%
Non-volatile linear silicone (6cS): 2%
Ethylhexyl methoxycinnamate: 7.5%
Disteardimonium hectorite: 1.4%
Lauryl PEG-9 polydimethylsiloxyethyl dimethicone: 1.5%
Isostearic acid: 1%
Alkyl acrylate/dimethicone copolymer: 0.5%
Diphenyldimethicone/vinyl diphenyldimethicone/silsesquioxane crosspolymer: 7%
Hydrophobized fine particulate zinc oxide: 7%
Hydrophobized fine particulate titanium oxide: 3%

*Example Formulation 8: makeup base (water-in-oil type)*

Volatile linear silicone (1.5cS): 70%
Ethylhexyl methoxycinnamate: 5%
PEG-9 polydimethylsiloxyethyl dimethicone: 1.5%
Isostearic acid: 0.5%
Polysilicone-22: 3%
Diphenyldimethicone/vinyl diphenyldimethicone/silsesquioxane crosspolymer: 6%
Hydrophobized fine particulate titanium oxide: 5%
Hydrophobized pigment-grade titanium oxide: 2%
Hydrophobized yellow iron oxide: 0.4%
Hydrophobized yellow iron oxide: 0.12%
Hydrophobized black iron oxide: 0.02%
Hydrophobized mica: appropriate amount
Antiseptic: appropriate amount
Ion-exchanged water: balance

*Example Formulation 9: makeup base (oil-based)*

Volatile linear silicone (1.5cS): balance
Ethylhexyl methoxycinnamate: 3%
Trimethylsiloxysilicate: 5%
PEG-9 polydimethylsiloxyethyl dimethicone: 1.8%
Isostearic acid: 0.8%
Diphenyldimethicone/vinyl diphenyldimethicone/silsesquioxane crosspolymer: 5%
Nylon 12: 3%
Hydrophobized fine particulate titanium oxide: 5%
Hydrophobized pigment-grade titanium oxide: 1%
Hydrophobized yellow iron oxide: 0.2%
Hydrophobized yellow iron oxide: 0.05%
Hydrophobized black iron oxide: 0.01%

*Example Formulation 10: emulsion (water-in-oil type)*

Volatile linear silicone (1.5cS): 65%
Decamethylcyclopentasiloxane: 5%
Non-volatile linear silicone (6cS): 3.5%
Disteardimonium hectorite: 1%
PEG-10 dimethicone: 3%
Isostearic acid: 1%
Adipic acid/neopentyl glycol crosspolymer: 3%

Diphenyldimethicone/vinyl diphenyldimethicone/silsesquioxane crosspolymer: 1%
Antiseptic: appropriate amount
Ion-exchanged water: balance
Dipropylene glycol: 1%
Na hyaluronate: appropriate amount

**Claims**

1. A cosmetic comprising a silicone oil in an amount of 50 mass % or more relative to the total mass of the cosmetic and having a viscosity of 10000 mPa·s or less (measured with a BL-type viscometer with a #3 rotor under measurement conditions of: 12 rpm, 1 minute, at 30°C), the cosmetic comprising:

   (a) a volatile linear silicone oil in an amount of 60 mass % or more relative to the total mass of the silicone oil; and
   (b) an elastic powder having a linear silicone oil absorption of not less than 40 g/100 g and not more than 125 g/100 g and a rheometer hardness (measured using SUN rheometer (CMPAC-II, available from SUN SCIENTIFIC CO., LTD)) of not less than 40 and not more than 300 in an amount of 1 to 10 mass % relative to the total mass of the cosmetic, and
   wherein the cosmetic contains an elastic powder having a linear silicone oil absorption higher than 125 g/100 g in an amount of not more than 3 mass % relative to the total mass of the cosmetic.

2. The cosmetic as claimed in claim 1, wherein the elastic powder comprises diphenyldimethicone/vinyl diphenyldimethicone/silsesquioxane crosspolymer, polysilicone-22, or adipic acid/neopentyl glycol crosspolymer.

3. The cosmetic as claimed in claim 1 or 2, wherein the cosmetic is a water-in-oil type emulsion cosmetic.

4. The cosmetic as claimed in claim 1 or 2, wherein the cosmetic is an oil-based cosmetic.

**Patentansprüche**

1. Kosmetikprodukt, das ein Silikonöl in einer Menge von 50 Gew.-% oder mehr in Bezug auf das Gesamtgewicht des Kosmetikprodukts umfasst und eine Viskosität von 10.000 mPa·s oder weniger aufweist (gemessen mit einem BL-Viskosimeter mit einem #3-Rotor unter folgenden Messbedingungen: 12 U/min, 1 Minute, bei 30 °C), wobei das Kosmetikprodukt folgendes umfasst:

   (a) ein flüchtiges lineares Silikonöl in einer Menge von 60 Gew.-% oder mehr in Bezug auf das Gesamtgewicht des Silikonöls; und
   (b) ein elastisches Pulver, das eine lineare Silikonölabsorption von nicht weniger als 40 g/100 g und nicht mehr als 125 g/100 g und eine Rheometerhärte (gemessen unter Verwendung eines SUN-Rheometers (CMPAC-II, erhältlich bei SUN SCIENTIFIC CO., LTD)) von nicht weniger als 40 und nicht mehr als 300 in einer Menge von 1 bis 10 Gew.-% in Bezug auf das Gesamtgewicht des Kosmetikprodukts aufweist, und
   wobei das Kosmetikprodukt ein elastisches Pulver enthält, das eine lineare Silikonölabsorption von mehr als 125 g/100 g in einer Menge von nicht mehr als 3 Gew.-% in Bezug auf das Gesamtgewicht des Kosmetikprodukts aufweist.

2. Kosmetikprodukt nach Anspruch 1, wobei das elastische Pulver Diphenyldimethicone/Vinyl Diphenyldimethicone/Silsesquioxane Crosspolymer, Polysilicone-22 oder Adipinsäure/Neopentyglykol Crosspolymer umfasst.

3. Kosmetikprodukt nach Anspruch 1 oder 2, wobei das Kosmetikprodukt ein Kosmetikprodukt vom Typ Wasser-in-Öl-Emulsion ist.

4. Kosmetikprodukt nach Anspruch 1 oder 2, wobei das Kosmetikprodukt ein Kosmetikprodukt auf Ölbasis ist.

**Revendications**

1. Produit cosmétique comprenant une huile de silicone en une quantité supérieure ou égale à 50 % en masse par rapport à la masse totale du produit cosmétique et ayant une viscosité inférieure ou égale à 10 000 mPa·s (mesurée avec un viscosimètre de type BL avec un rotor n°3 dans des conditions de mesure de : 12 tr/min, 1 minute, à 30 °C), le produit cosmétique comprenant :

(a) une huile de silicone linéaire volatile en une quantité supérieure ou égale à 60 % en masse par rapport à la masse totale de l'huile de silicone ; et
(b) une poudre élastique ayant une absorption d'huile de silicone d'au moins 40 g/100 g et d'au plus 125 g/100 g et une dureté au rhéomètre (mesurée en utilisant un rhéomètre SUN (CMPAC-II, disponible auprès de SUN SCIENTIFIC CO., LTD)) d'au moins 40 et d'au plus 300 en une quantité de 1 à 10 % en masse par rapport à la masse totale du produit cosmétique, et
dans lequel le produit cosmétique contient une poudre élastique ayant une absorption d'huile de silicone linéaire supérieure à 125 g/100 g en une quantité d'au plus 3 % en masse par rapport à la masse totale du produit cosmétique.

2. Produit cosmétique selon la revendication 1, dans lequel la poudre élastique comprend un polymère réticulé de diphényldiméthicone/diphényldiméthicone vinylique/silsesquioxane, de la polysilicone-22, ou un polymère réticulé d'acide adipique/néopentylglycol.

3. Produit cosmétique selon la revendication 1 ou 2, dans lequel le produit cosmétique est un produit cosmétique en émulsion de type eau dans huile.

4. Produit cosmétique selon la revendication 1 ou 2, dans lequel le produit cosmétique est un produit cosmétique huileux.

**EP 3 357 488 B1**

**REFERENCES CITED IN THE DESCRIPTION**

**Patent documents cited in the description**

- JP H8259417 B

- JP 2000229838 A

22